# EUROPEAN PATENT APPLICATION

(11) **EP 1 656 912 A1**
(43) Date of publication of application: **17.05.2006**
(21) Application number: 05024598.4
(22) Date of filing: 10.11.2005
(51) Int. Cl.: A61F 5/04

(54) **Cervical traction device**

(30) Priority: 12.11.2004 US 986657
(71) Applicant: CARE REHAB & ORTHOPEDIC PRODUCTS, INC., McLean VA 22102 (US)
(72) Inventor: Hunt, Christian, McLean, VA 22102 (US); Boghosian, Ara, Alexandria, VA 22312 (US)
(74) Representative: Herzog, Markus

(57) **Abstract**

A traction device (100), comprising a stationary support (104) and a movable carriage (106) mounted on the stationary support. A mechanism is provided for moving the moveable carriage between a first position and a second position. A wedge system (118) is mounted to the moveable carriage and a stand is adjustably mounted to the stationary support. The stand (101) provides different adjustable angles for the wedge system.

## Description

### CROSS REFERENCE TO RELATED APPLICATIONS

This application claims priority to U.S. Provisional Application Serial No. 60/519,793, filed on November 14, 2003, which is incorporated herein by reference.

### Field of the Invention

The invention generally relates to a cervical traction device and, more particularly, to a cervical traction device having adjustable wedges and stand.

### BACKGROUND OF THE INVENTION

Traction devices are used to relieve pressure on inflamed or enlarged nerves. Cervical and lumbar or spinal traction are the most common type of devices. When correctly performed, the traction devices can relieve pain in the neck and the spine by, for example, straightening the curvature of the spine or stretching of the spinal and cervical musculature.

Portable traction devices are now becoming very popular for in home traction devices. This allows patients to perform traction therapy without leaving their homes, or expending large sums of money for a healthcare provider or physical therapist.

Known portable traction devices include pneumatic cylinders controllable by a hand pump. In one known device, a special pressure activated seal is used in the pneumatic device to purportedly provide a static traction force during a full therapy section. However, such traction device is expensive. Also, known traction devices include wedges which rotate. This rotation, though, may make it difficult to obtain a desired adjustment of the wedges. These same devices do not have any mechanism to adjust the angle of the entire device, or other mechanisms to ensure ease of adjustment with the confidence that such adjustment will remain in a locked position.

### BRIEF SUMMARY OF THE INVENTION

The invention is directed to a portable traction device. The portable traction device has stand that allows adjustment of the device at various angles. The portable traction device also includes a mechanism to allow linear adjustment of the wedge system, which includes a locking system.

The cervical traction device typically includes a carriage slidable along a portion of a supporting track. A pneumatic device moves the carriage along the supporting track. The supporting track and carriage are mounted, slidably, in embodiments, to a stand. The pneumatic device may also be a hydraulic cylinder, an electric motor, or a spring-loaded device. The stand can be locked in place such that the device can be adjusted to a plurality of different angles.

### BRIEF DESCRIPTION OF THE DRAWINGS

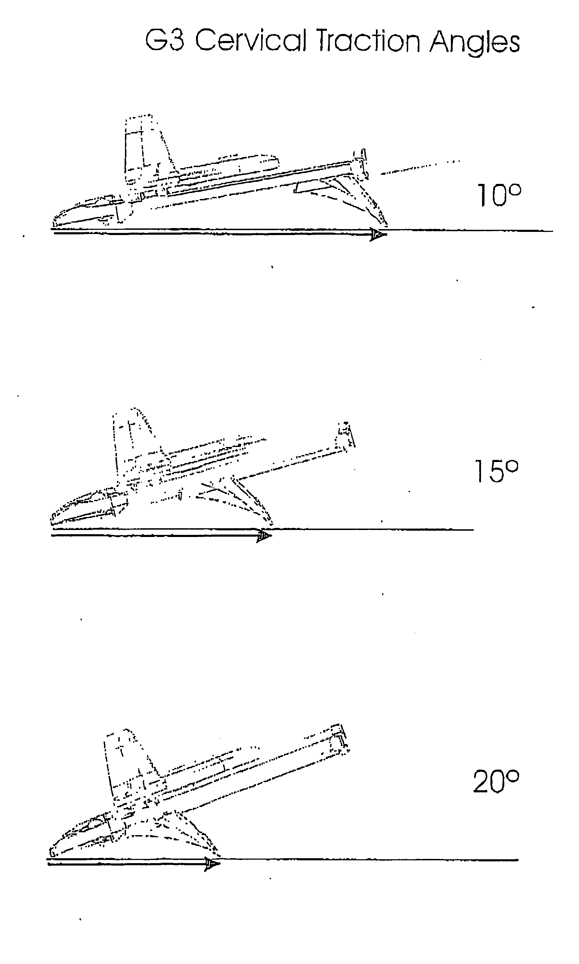

Figures 1A-1C show different adjustable angles of the cervical traction device in accordance of the invention;

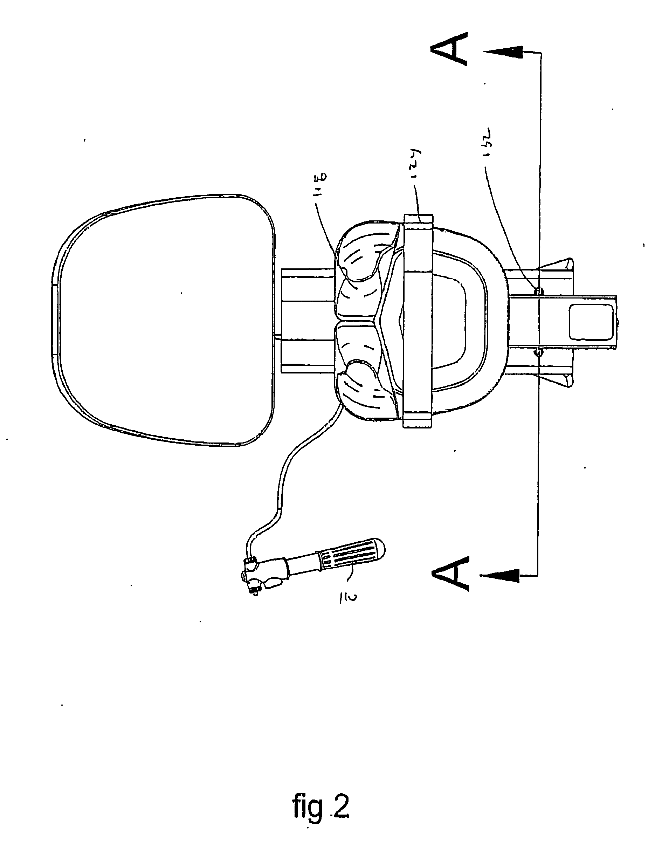

Figure 2 shows a top view of the cervical traction device in accordance of the invention;

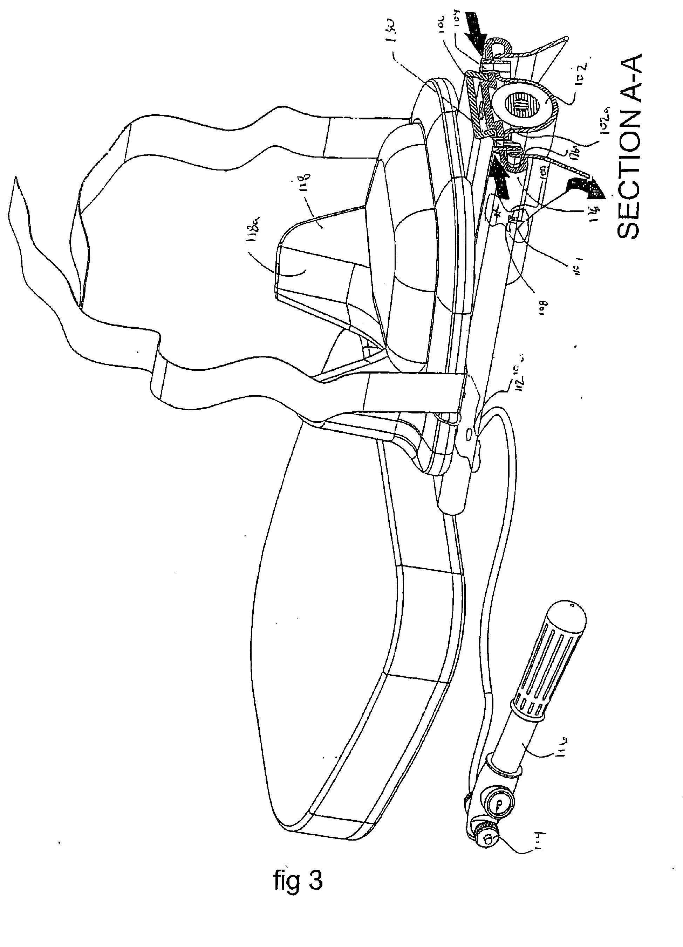

Figure 3 shows a perspective view of the cervical traction device in accordance of the invention;

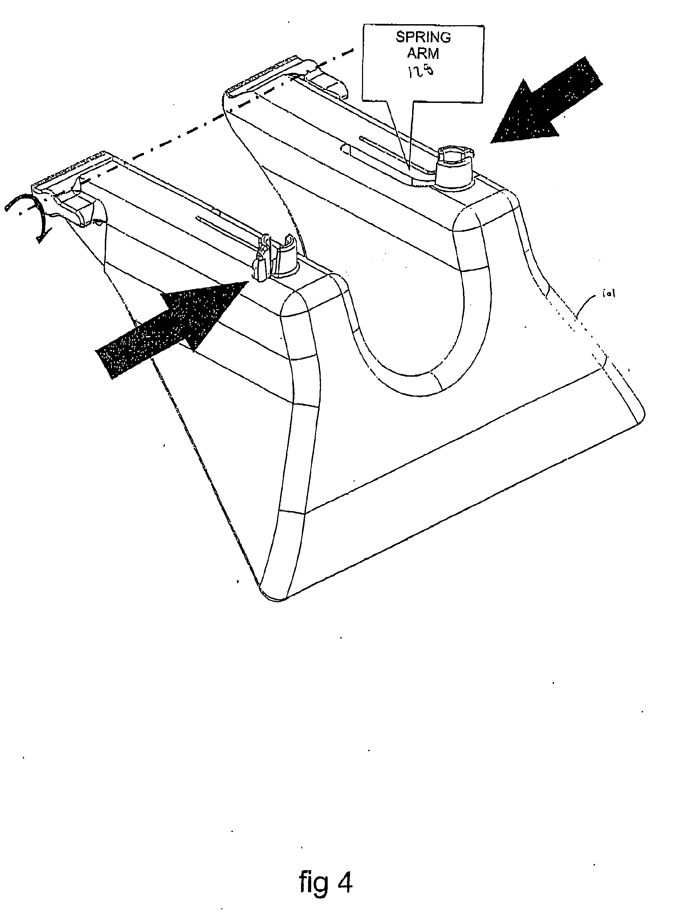

Figure 4 shows a the stand of the cervical traction device in accordance of the invention;

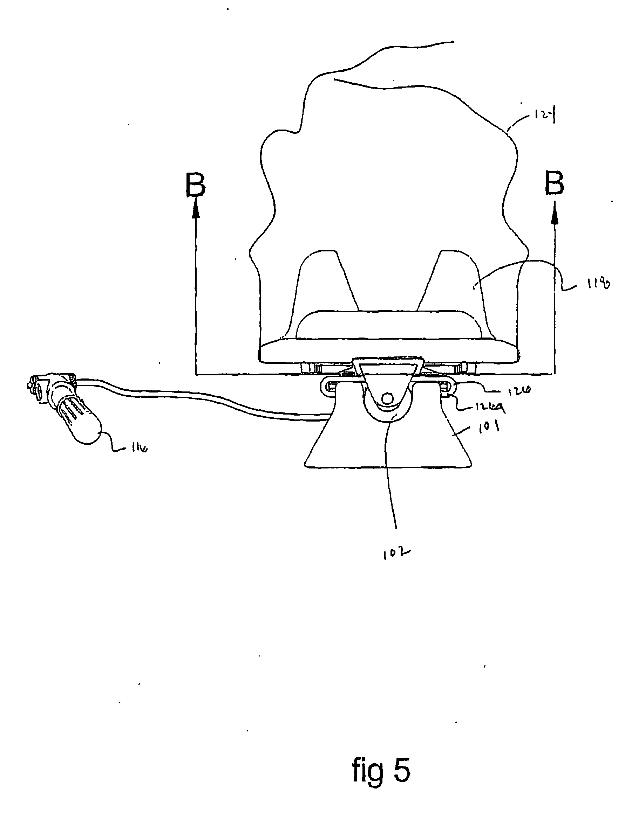

Figure 5 shows a rear view of the cervical traction device in accordance of the invention;
Figure 6 shows a top view of the cervical traction device in accordance of the invention;
Figure 7 shows a bottom view of the cervical traction device and wedge insert in accordance of the invention; and
Figures 8A and 8B show an anti rotation rib in accordance with the invention.

### DETAILED DESCRIPTION OF EMBODIMENTS OF THE INVENTION

The invention is directed to a cervical traction device. The cervical traction device is adjustable at different angles and also includes adjustable wedge supports, in addition to other features,

### Cervical Traction Device

Referring now to the figures, a schematic diagram of the cervical traction device is shown. The cervical traction device 100 includes several different adjustable angles such as 10 degrees, 15 degrees and 20 degrees. It should be understood, though, that other angles of adjustment, with relation to the floor, are also contemplated by the invention, and that the adjustable angles shown herein are provided for illustrative purposes. The cervical traction device is adjustable via a stand 101 positioned underneath a movable carriage mounted on a stationary track. The carriage is slidably mounted along a support track via a pneumatic cylinder structure. The carriage includes a head rest or support pad.

In the cervical traction configuration, a pneumatic cylinder 102 is mounted between a support structure 104 and the movable carriage 106. The pneumatic cylinder 102 is, in one embodiment, a single-acting pneumatic cylinder with a piston 109 having a diameter slightly smaller than the inside diameter of the cylinder housing 102A. A slot 108 extending around the outside circumference of the piston 109 contains a compression seal 110. In this design, the compression seal 110 is a conventional o-ring seal that remains in contact or engaged with the inner wall of the cylinder housing, regardless of the application of pressure within the cylinder housing 102A. The o-ring provides a uniform and constant frictional force on the cylinder wall.

While application of pressure within the housing may negligibly deform the o-ring, it has no overall affect on the performance of the system since the seal remains in constant engagement with the wall of the cylinder. The o-ring does not relax and move away from the cylinder wall upon the removal of pressure. An air inlet 112 is provided at an end of the cylinder housing 102 for providing pressurized air to the cylinder. A manual pressure relief mechanism 114 may be used with a pump 116 for providing and releasing pressurized air to and from the pneumatic cylinder. A pressure release valve may also be used, as well as a gauge to show the pressure.

Other types of systems may also be used to move the carriage such as a ratchet type assembly, a linear actuator, an accordion type device and the like. Other types of cylinders, known to those of skill in the art, may also be used with the moveable carriage.

As shown, the cervical traction device includes an wedge system 118 for applying a therapeutic traction force to the occipital areas on a patent's head and neck. The wedges 118 are mounted to the carriage 106. In this configuration, the wedges do not rotate. That is, the wedges remain stationary (i) when the patient initially places his/her head between the wedges and (ii) during the application of traction forces. The wedges may have a concave engaging surface 118A.

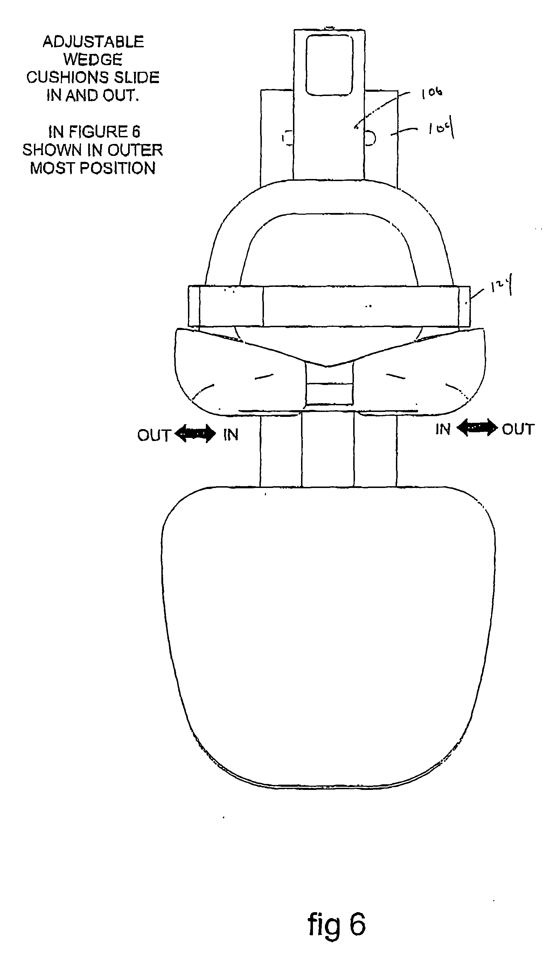

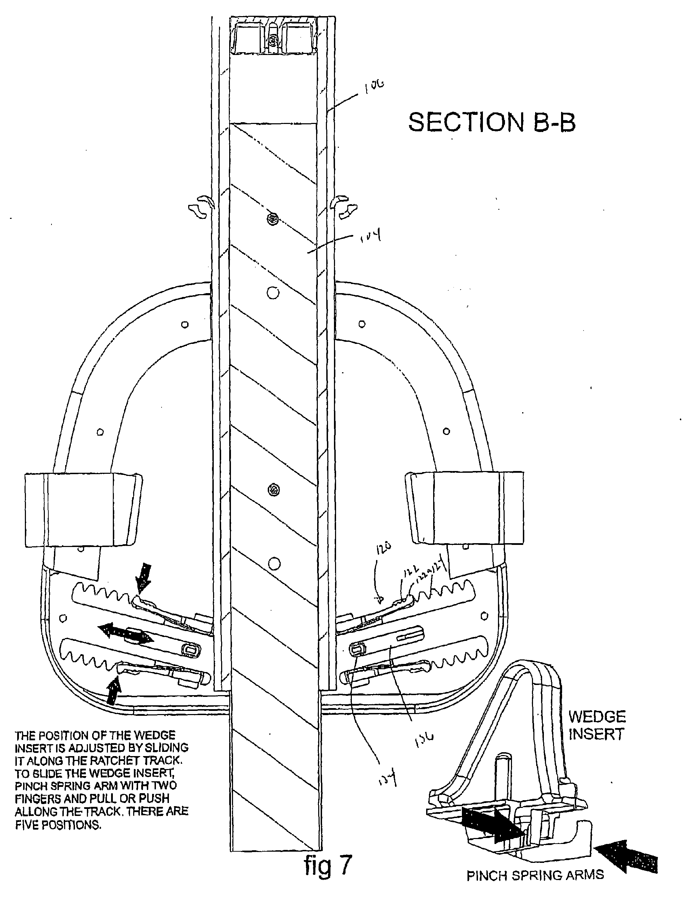

The wedges, though, may move or slide on the carriage and are adjustable by a pinch spring arm mechanism 120. As shown in Figure 7, for example, the wedges can slide in several different positions by providing a ratchet type track in/on the moveable carriage. The spring arms 122 will engage the ratchet 124 at different positions via a tabbed or protruding portion 122A. The spring arms 122 may have tabs so that the user can squeeze them together to disengage from a ratchet portion of the ratchet track, and then move the wedges in or out as shown by the arrows in Figure 6. The movement of the wedges may be in a somewhat linear manner across the carriage. While a restraining belt 124 is used in the cervical traction device, it may be excluded from the cervical traction device.

The track includes a system for allowing adjustment of the stand so that several angles can be achieved. The stand 101 may be wedge shaped, for example, and may include features in order to slide around the mounted cylinder assembly. In one implementation, the track 126 includes turn ends 126A which form a rail. The rail accommodates a slide mechanism 130 on the stand 101 such that the stand can slide within the track 126. A spring arm, hook or other type of mechanism 128 may then be used to "lock" the stand into position. In one implementation, the track 126 will have apertures or holes 132 which will accommodate the spring arm, hook or other type of mechanism. The spring arm can be used to lock within the holes.

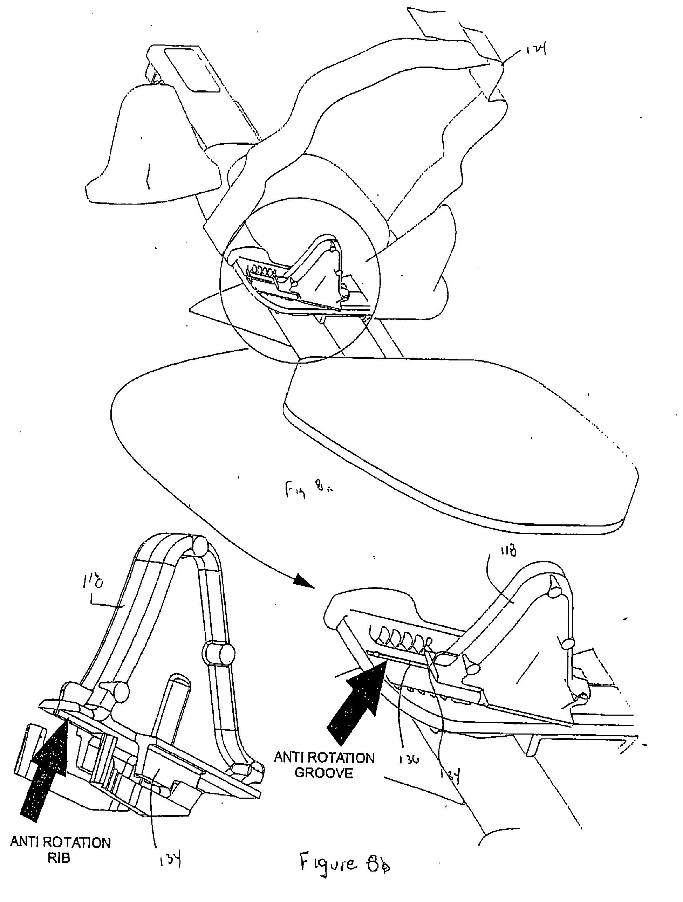

The device also includes an anti-rotation rib 134 and corresponding slot or slit 136 for preventing rotation of the wedge. The slot 136 is in the carriage and is, in one implementation, proximate to the ratchet track. The rib 134 communicates with the slot 136 while allowing the wedges to still slide in and out. The wedges can be mounted onto the frame as shown in Figures 8A and 8B.

### Operation of the Cervical

### Traction Device

To use the cervical traction device, the cylinder assembly is initially at atmospheric pressure. The back of the user is placed on a support surface so that the neck is cradled by the neck supports, i.e., wedges. These supports remain stationary. A restraining belt may be used about the patient's head to ensure that it remains stationary during treatment. The user then pumps air into the cylinder using the air pump. The patient increases the traction force by manually operating the pump or decreases the traction force by manually pressing the pressure relief mechanism. As air is pumped into the cylinder, the piston will extend from the cylinder housing and the carriage will begin to separate from the support structure. In this manner, a traction force will be provided to the user.

Prior to using the device, the patient or user can adjust the angle of the cervical traction device using the stand assembly. The neck supports, i.e., wedges, can also be adjusted.

While the invention has been described in terms of embodiments, those skilled in the art will recognize that the invention can be practiced with modification.

A traction device, comprising a stationary support and a movable carriage mounted on the stationary support. A mechanism is provided for moving the moveable carriage between a first position and a second position. A wedge system is mounted to the moveable carriage and a stand is adjustably mounted to the stationary support. The stand provides different adjustable angles for the wedge system.

## Claims

1. A device, comprising:
a stationary support;
a movable carriage mounted to the stationary support;
a means for moving the moveable carriage between a first position and a second position;
a wedge system mounted to the moveable carriage; and
a stand adjustably mounted to the stationary support, the stand providing different adjustable angles for the wedge system.

2. The device of claim 1, wherein the stand is a wedged shaped stand.

3. The device of claim 1, wherein the stationary support includes a turned end which forms a rail to accommodate portions of the stand and allow adjustment thereof.

4. The device of claim 3, wherein the rail accommodates a slide mechanism of the stand.

5. The device of claim 1, wherein the stand includes a locking mechanism to lock the stand to the stationary support in one of the adjustable positions.

6. The device of claim 1, wherein the locking mechanism includes a spring arm or hook and apertures or holes which accommodate the spring arm or hook.

7. The device of claim 1, wherein the means for moving is a pneumatic cylinder mounted between a support structure of the stationary support and the movable carriage.

8. The device of claim 7, wherein the pneumatic cylinder is a single-acting pneumatic cylinder with a piston having a diameter slightly smaller than the inside diameter and an o-ring disposed about a slot in the piston.

9. The device of claim 1, wherein the wedges do not rotate.

10. The device of claim 1, wherein the wedges remain stationary (i) when a patient initially places his/her head between the wedges and (ii) during an application of traction forces.

11. The device of claim 1, wherein the wedges are adjustable by a pinch mechanism.

12. The device of claim 11, wherein the pinch mechanism includes a ratchet track in the moveable carriage and spring arms extending from the wedges and engaging the ratchet track at different positions via a tabbed or protruding portion.

13. The device of claim 1, wherein the wedges are moveable in a substantially linear motion across the moveable carriage.

14. The device of claim 1, further comprising anti-rotation device to prevent rotation of the wedges.

15. The device of claim 14, wherein the anti-rotation device is a rib extending from the wedges and corresponding slot in the moveable carriage.
